# EUROPEAN PATENT APPLICATION

(11) **EP 2 425 831 A1**
(43) Date of publication of application: **07.03.2012**
(21) Application number: 10769800.3
(22) Date of filing: 28.04.2010
(51) Int. Cl.: A61K 31/17, A61K 31/4015, A61K 31/4166, A61P 17/00, A61P 17/06, A61P 35/00, A61P 37/02, A61P 37/08, A61P 43/00, C07D 207/27, C07D 233/34, C07D 233/40

(54) **SCCA-1 PRODUCTION INHIBITOR HAVING A CARBOXAMIDE DERIVATIVE AND/OR A SALT THEREOF AS AN ACTIVE INGREDIENT**

(30) Priority: 28.04.2009 JP 2009110112
(71) Applicant: Shiseido Co., Ltd., Tokyo 104-8010 (JP)
(72) Inventor: KANEKO, Maki, Yokohama-shi Kanagawa 224-8558 (JP); HIRUMA, Takuya, Yokohama-shi Kanagawa 224-8558 (JP); SUETSUGU, Masaru, Yokohama-shi Kanagawa 224-8558 (JP); KATAGIRI, Chika, Yokohama-shi Kanagawa 236-8643 (JP); IIDA, Toshii, Yokohama-shi Kanagawa 224-8558 (JP); ONODERA, Tomoko, Yokohama-shi Kanagawa 236-8643 (JP)
(74) Representative: Hart-Davis, Jason
(86) International application number: PCT/JP2010/057615
(87) International publication number: WO 2010/126102

(57) **Abstract**

A SCCA-1 production inhibitor, comprising as an active ingredient, at least one carboxyamide derivative selected from the group consisting of compounds represented by the following formula (I): 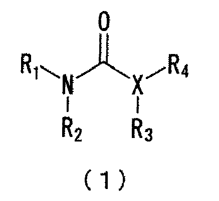 wherein X is CR₅ or N, R₁, R₄ and R₅ each independently represent hydrogen, C₁-C₄ alkyl or a C₁-C₆ hydroxyalkyl group having 1-5 hydroxy groups, R₂ and R₃ each independently represent hydrogen, C₁-C₄ alkyl or a C₁-C₆ hydroxyalkyl group having 1-5 hydroxy groups, or each represents a group -(CH₂)ₙ-, wherein n represents an integer of 1 or 2, and may form a 5- to 6-membered ring together with the atoms to which they are bonded and with the carbonyl group, allantoin, and salts thereof.

SEQUENCE LISTING

<110>  SHISEIDO COMPANY, LTD.

<120>  SCCA-1 Production Inhibitor Comprising Carboxyamide Derivative and/or
Salt Thereof as Active Component

<130>  Y587-PCT

<150>  JP2009-110112
<151>  2009-04-28

<160>  6

<170>  PatentIn version 3.1

<210>  1
<211>  17
<212>  DNA
<213>  Artificial

<220>
<223>  Human SCCA-1 Forward Primer

<400>  1
gtgctatctg gagtcct                                                    17


<210>  2
<211>  16
<212>  DNA
<213>  Artificial

<220>
<223>  Human SCCA-1 Reverse Primer

<400>  2
ctgttgttgc cagcaa                                                     16


<210>  3
<211>  17
<212>  DNA
<213>  Artificial

<220>
<223>  Human SCCA-1 Probe

<400>  3
catcacctac ttcaact                                                    17


<210>  4
<211>  19
<212>  DNA
<213>  Artificial

<220>
<223>  Human GAPDH Forward Primer

<400>  4
gaaggtgaag gtcggagtc                                                  19


<210>  5

<211>  20
<212>  DNA
<213>  Artificial

<220>
<223>  Human GAPDH Reverse Primer

<400>  5
gaagatggtg atgggatttc                                                    20


<210>  6
<211>  22
<212>  DNA
<213>  Artificial

<220>
<223>  Human GAPDH Probe

<400>  6
aggctgagaa cgggaagctt gt                                                 22

## Description

### Technical Field

The present invention relates to a Squamous Cell Carcinoma Antigen-1 (hereunder abbreviated as "SCCA-1") production inhibitor comprising a carboxyamide derivative and/or a salt thereof as an active ingredient.

### Background Art

Squamous cell carcinoma antigen (SCCA), an antigen expressed in squamous carcinoma cells, is found in high blood concentration in squamous cell carcinoma of the uterine cervix, lungs, esophagus and skin, and it is commonly used for diagnosis of squamous cell carcinoma (Non-patent document 1: H. Kato et al. Cancer 40:1621-1628 (1977), Non-patent document 2: N. Mino et al. Cancer 62: 730-734 (1988)).

SCCA is known to have accelerated expression not only in squamous cell carcinoma, but according to research by the present inventors, also in the upper layer of psoriatic epidermis (Non-patent document 3: Takeda A. et al, J. Invest. Dermatol. (2002) 118(1), 147-154). Psoriasis is a type of skin disease with high frequency, and may take the form of chronic, relapsing inflammatory parakeratosis characterized by abnormal proliferation and differentiation of epidermal cells and infiltration of inflammatory cells. Psoriasis is believed to occur as a result of genetic disposition and several environmental factors (Non-patent document 4: Hopso-Havu et al. British Journal of Dermatology (1983) 109, 77-85).

SCCA is encoded by the two genes SCCA-1 and SCCA-2 situated in tandem on chromosome 18q21.3. The proteins encoded thereby, SCCA-1 and SCCA-2, are both proteins with molecular weights of approximately 45,000, and while they have very high homology, their amino acid sequences differ at the reactive sites, whereby their different functions are presumably exhibited (Non-patent document 5: Schick et al. J. Biol. Chem. (1997) 27213, 1849-55).

The present inventors have conducted research with the aim of elucidating the physiological mechanism of SCCA-1 and SCCA-2 in the epidermis, and as a result we have obtained the knowledge that both SCCA proteins are anti-apoptotic factors having effects of inhibiting cellular apoptosis (Patent document 1: Japanese Unexamined Patent Publication No. 2005-281140).

The present inventors have also conducted research with the aim of evaluating the sensitivity of skin, using SCCA-1 expression in particular as an index, and as a result we have obtained the knowledge that SCCA-1 expression is increased 16-fold in atopic xeroderma, 90-fold in light-exposed skin, 232-fold in pollen hypersensitive allergenic skin and 466-fold in psoriatic skin, compared to controls (Japanese Patent Application No. 2006-075024).

The present inventors, having further researched the relationship between cell proliferation and SCCA, and especially SCCA-1, have also obtained the knowledge that:
- cell proliferation is activated in high SCCA-expressing mice,
- acanthosis is seen in high SCCA-expressing mice,
- a correlation exists between cell proliferation and SCCA-1 expression in high SCCA-1-expressing cell lines, and
- cell proliferation activity is reduced in SCCA knock-down cell lines (Japanese Patent Application No. 2007-279024 and Journal of Cell Biology, 172(7), 983-990 (2006)).

Thus, a substance that effectively inhibits production of SCCA, and especially SCCA-1, is believed to be useful for prevention and/or treatment of diseases associated with abnormal cell proliferation, and diseases associated with increased SCCA-1 production.

### Citation List

### Patent Literature

[Patent document 1] Japanese Unexamined Patent Publication No. 2005-281140

### Non-patent literature

[Non-patent document 1] Cancer 40:1621-1628 (1977)
[Non-patent document 2] Cancer 62: 730-734 (1988)
[Non-patent document 3] J. Invest. Dermatol. 118(1), 147-154(2002)
[Non-patent document 4] British Journal of Dermatology 109, 77-85(1983)
[Non-patent document 5] J. Biol. Chem. 27213, 1849-55(1997)
[Non-patent document 6] Journal of Cell Biology, 172(7), 983-990 (2006)

### DISCLOSURE OF THE INVENTION

### Problems to be Solved by the Invention

The present inventors, as a result of focusing on the relationship between cell proliferation and SCCA-1 and screening a large variety of medicinal agents, have found that certain carboxyamide derivatives and/or their salts significantly inhibit production of SCCA-1. The present inventors have therefore concluded that these carboxyamide derivatives and/or their salts are highly useful for prevention and/or treatment of diseases associated with abnormal cell proliferation and diseases associated with increased SCCA-1 production, due to their inhibitory action on SCCA-1 production, and the invention has thereupon been completed.

### Means for Solving the Problems

The present invention encompasses the following aspects.
[1] A SCCA-1 (Squamous Cell Carcinoma Antigen 1) production inhibitor, comprising as an active ingredient, at least one carboxyamide derivative selected from the group consisting of compounds represented by the following formula (I): 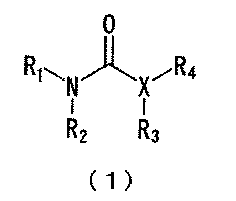 (wherein X is CR₅ or N,
   R₁, R₄ and R₅ each independently represent hydrogen, C₁-C₄ alkyl or a C₁-C₆ hydroxyalkyl group having 1-5 hydroxy groups,
   R₂ and R₃ each independently represent hydrogen, C₁-C₄ alkyl or a C₁-C₆ hydroxyalkyl group having 1-5 hydroxy groups, or each represents a group -(CH₂)ₙ-, wherein n represents an integer of 1 or 2, and may form a 5- to 6-membered ring together with the atoms to which they are bonded and with the carbonyl group),
   allantoin, and salts thereof.
[2] A SCCA-1 production inhibitor according to claim 1, wherein the carboxyamide derivative according to [1] above is one of the following: 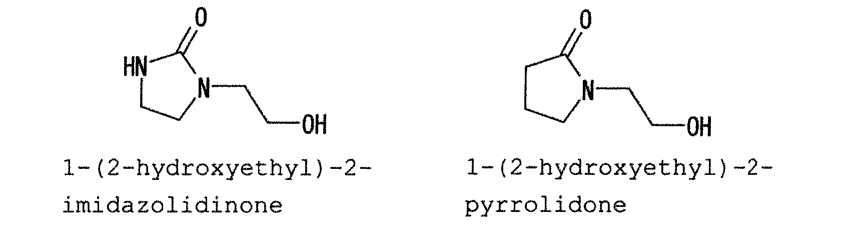 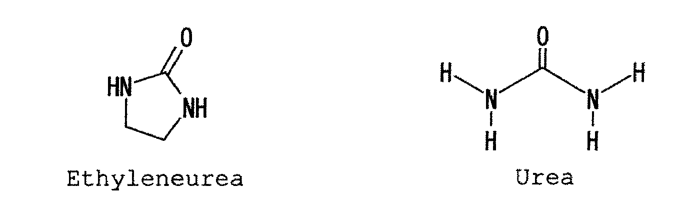 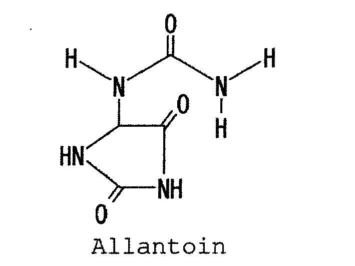 or a salt thereof.
[3] A pharmaceutical composition for prevention and/or treatment of a disease associated with abnormal cell proliferation, comprising the carboxyamide derivative according to [1] or [2] and/or a salt thereof as an active ingredient.
[4] A pharmaceutical composition for prevention and/or treatment of acanthosis, comprising a carboxyamide derivative according to [1] or [2] and/or a salt thereof as an active ingredient.
[5] A pharmaceutical composition for treatment of a disease selected from the group consisting of malignant tumors (carcinoma) and precancerous conditions, such as basal cell carcinoma (BCC), squamous cell carcinoma (SCC), Bowen's disease, pilomatricoma (calcifying epithelioma), seborrheic keratosis and actinic keratosis (solar keratosis), lichen planus-like keratosis, benign lichenoid keratosis, acrochordon (cutaneous tag), psoriatic conditions such as pustular psoriasis, psoriasis vulgaris, xeroderma pigmentosum (XP), atopic dermatitis, erythematosus including systemic lupus erythematosus (SLE) and discoid lupus erythematosus (DLE), porokeratosis, moles or verrucas such as inflammatory linear verrucous epidermal naevus (ILVEN), and benign keratosis such as hyperplasia, comprising a carboxyamide derivative according to [1] or [2] and/or a salt thereof as an active ingredient.

### Brief Description of the Drawings

Fig. 1 shows the SCCA-1 expression-inhibiting effects of different carboxyamide derivatives in a cell line.
Fig. 2 shows the SCCA-1 expression-inhibiting effect of allantoin in a cell line.
Fig. 3 shows the SCCA-1 expression-inhibiting effect of urea in a cell line.

### Best Mode for Carrying Out the Invention

A carboxyamide derivative and/or its salt according to the invention is highly useful for inhibiting production of SCCA-1. Specifically, it was found that addition of a carboxyamide derivative to culture medium during culturing of human keratinocytes significantly inhibits production of SCCA1. By inducing abnormal proliferation of epidermal cells and culturing a three-dimensional skin model with notably increased SCCA1 expression in carboxyamide derivative-containing medium, it was confirmed that acanthosis can be prevented and ameliorated. In addition, it was confirmed that by continuous application of the carboxyamide derivative onto human skin, SCCA1 levels can be significantly reduced in the applied sections.

Furthermore, upon examining the effect of carboxyamide derivatives and/or their salts according to the invention on human skin, they were found to be useful for inhibiting accelerated thickening of the epidermis of human skin.

Thus, by inhibiting production of SCCA-1, the carboxyamide derivatives and/or their salts according to the invention are useful for prevention and/or treatment of diseases associated with abnormal proliferation of cells, including malignant tumors and precancerous conditions such as basal cell carcinoma, squamous cell carcinoma, Bowen's disease, pilomatricoma (calcifying epithelioma), seborrheic keratosis and actinic keratosis (solar keratosis), lichen planus-like keratosis, benign lichenoid keratosis, acrochordon, psoriatic conditions such as pustular psoriasis and psoriasis vulgaris, xeroderma pigmentosum, atopic dermatitis, erythematosus such as systemic lupus erythematosus and discoid lupus erythematosus, porokeratosis, moles and verrucas including inflammatory linear verrucous epidermal naevus, and benign keratosis with hyperplasia.

When the carboxyamide derivative represented by formula (I) of the invention is a known substance, it may be easily synthesized by a known method or easily purchased as a commercial product, or if it is a novel compound, for example, it may be easily synthesized by a method known to those skilled in the art.

The carboxyamide derivative represented by formula (I) of the invention may also be converted to an inorganic salt or organic salt by a known method. There are no particular restrictions on salts to be used for the invention, and examples include inorganic salts such as hydrochlorides, sulfuric acid salts, phosphoric acid salts, hydrobromic acid salts, sodium salts, potassium salts, magnesium salts, calcium salts and ammonium salts. Organic salts include acetic acid salts, lactic acid salts, maleic acid salts, fumaric acid salts, tartaric acid salts, citric acid salts, methanesulfonic acid salts, p-toluenesulfonic acid salts, triethanolamine salts, diethanolamine salts and amino acid salts.

The SCCA-1 production inhibitor and pharmaceutical composition of the invention comprise a carboxyamide derivative and/or its salt, and a pharmaceutically acceptable excipient and/or carrier. The pharmaceutical composition comprises a carboxyamide derivative and/or its salt in an amount effective to exhibit the function, and the content is, for example, preferably 0.001-20.0 mass%, more preferably 0.01-10.0 mass% and most preferably 0.2-10.0 mass% of the total pharmaceutical composition, although this will vary depending on the purpose of use of the pharmaceutical composition. When the carboxyamide derivative and/or its salt is to be used in admixture, the upper limit for the total content is preferably no greater than 20.0 mass% and even more preferably no greater than 10.0 mass%.

The SCCA-1 production inhibitor and pharmaceutical composition of the invention may be in various dosage forms depending on the purpose, and for example, it may be administered in oral form such as a tablet, coated tablet, sugar-coated tablet, hard or soft gelatin capsule, solution or suspension, in enteral form such as a suppository, in a parenteral form such as an injection, or in external application form such as a patch, ointment, cream or latex.

The SCCA-1 production inhibitor and pharmaceutical composition of the invention may contain a pharmaceutically acceptable carrier as an appropriate inorganic or organic solid or solution, for example, as desired, together with the active ingredient. For example, it may contain a diluent (lactose, dextrose, saccharose, mannitol, sorbitol, cellulose or the like), a lubricant (silica, talc, or stearic acid or a salt thereof such as magnesium stearate or calcium stearate), and/or polyethylene glycol. A tablet may contain a binder (aluminum silicate magnesium, starch, gelatin, tragacanth, methylcellulose, sodium carboxymethylcellulose and/or polyvinylpyrrolidone, or the like), and if desired a disintegrator (starch, agar, alginic acid or a salt thereof, and/or an expandable mixture or the like), an absorbent, a coloring agent, a flavoring and/or a sweetener, for example.

The SCCA-1 production inhibitor and pharmaceutical composition of the invention may also contain a preservative, solubilizing agent, stabilizer, moistening agent, emulsifier, sweetener, coloring agent or flavoring agent, a salt for osmotic pressure variation, a buffering agent, a coating agent or an antioxidant. The SCCA-1 production inhibitor and pharmaceutical composition of the invention may further contain substances with therapeutic value, such as active ingredients other than the carboxyamide derivative and/or its salt according to the invention.

The method and amount for use of the SCCA-1 production inhibitor and pharmaceutical composition of the invention may be varied within a wide range, and can be decided by a method known to a person in the field. The method and amount for use are adjusted as necessary for each individual specific case, in consideration of the route of administration, the symptoms to be treated and the patient to be treated. The dosage may vary according to the purpose of use and the dosage form of the drug composition and on the body weight and body surface area of the patient, but it is preferably a dosage of 0.1 µg-10,000 mg and even more preferably 100 µg-1000 mg, per day. It may be administered all at once or in divided doses, and the method of administration may be oral or injection, or application.

### [Example 1]

SCCA-1 expression-inhibiting effects of carboxyamide derivatives in cell line:

Human keratinocytes were cultured, and carboxyamide derivatives at different concentrations were added at 60-70% confluency. The carboxyamide derivatives used were the following:

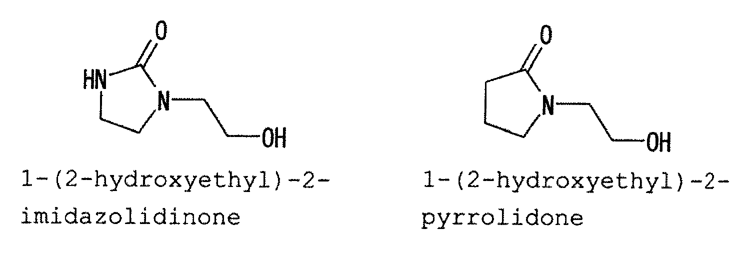

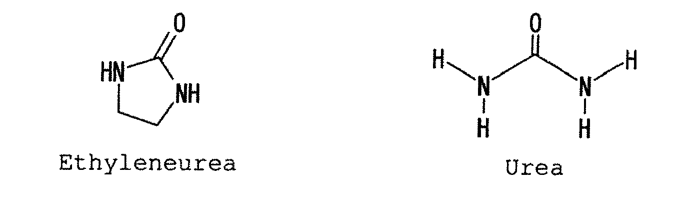

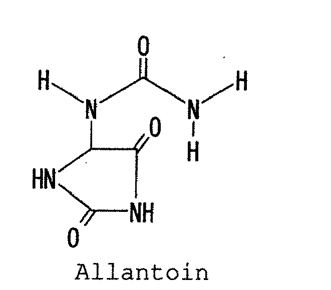

After 24 hours, the RNA was obtained, and then it was subjected to a quantitative PCR analysis by ABI PRISM 7900HT Sequence Detector System (Taqman PE) (Applied Biosystems) with using a primer/probe combination shown below, thereby the amount of SCCA gene expression was measured (using G3PDH as the internal standard).
H uman SCCA1
   Forward primer: 5'-GTGCTATCTGGAGTCCT-3' (SEQ ID NO: 1)
   Reverse primer: 5'-CTGTTGTTGCCAGCAA-3' (SEQ ID NO: 2)
   Probe: 5'-CATCACCTACTTCAACT-3' (SEQ ID NO: 3)
H uman G3PDH
   Forward primer: 5'-GAAGGTGAAGGTCGGAGTC-3' (SEQ ID NO: 4)
   Reverse primer: 5'-GAAGATGGTGATGGGATTTC-3' (SEQ ID NO: 5)
   Probe: 5'-AGGCTGAGAACGGGAAGCTTGT-3' (SEQ ID NO: 6)

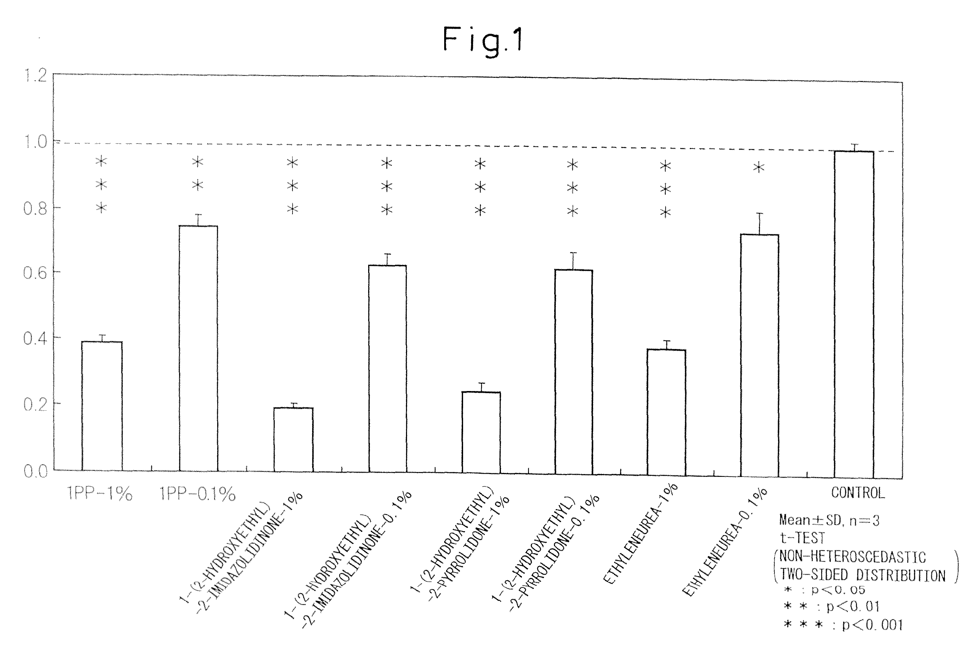

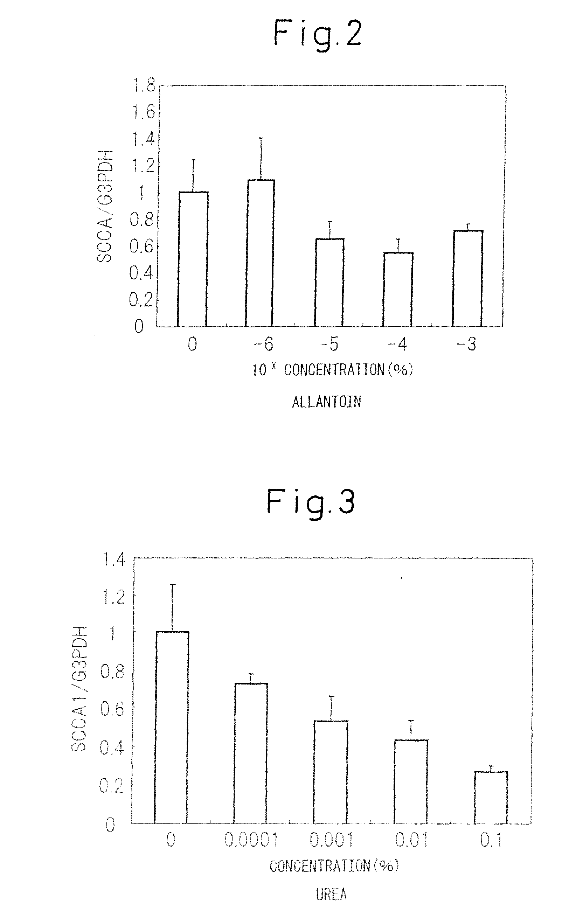

Fig. 1 shows the results with addition of the compound 1-(2-hydroxyethyl)-2-imidazolidinone, 1-(2-hydroxyethyl)-2-pyrrolidone or ethyleneurea. As a negative control, no drug was added, and as a positive control there was added 1-piperidinepropionic acid (1-PP) (Japanese Patent Application No. 2008-903571), which is known to have a gene expression-inhibiting effect on SCAA-1 and SCAA-2. As shown in the graph, a significant dose-dependent reduction in SCCA-1 gene expression was seen. Fig. 2 and Fig. 3 show the results of adding allantoin and urea, respectively, and similarly, a significant dose-dependent reduction in SCCA-1 gene expression was seen.

## Claims

1. A SCCA-1 (Squamous Cell Carcinoma Antigen 1) production inhibitor, comprising as an active ingredient, at least one carboxyamide derivative selected from the group consisting of compounds represented by the following formula (I): 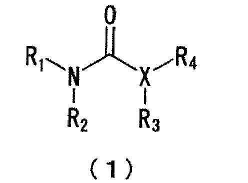 wherein X is CR₅ or N,
R₁, R₄ and R₅ each independently represent hydrogen, C₁-C₄ alkyl or a C₁-C₆ hydroxyalkyl group having 1-5 hydroxy groups,
R₂ and R₃ each independently represent hydrogen, C₁-C₄ alkyl or a C₁-C₆ hydroxyalkyl group having 1-5 hydroxy groups, or each represents a group -(CH₂)ₙ-, wherein n represents an integer of 1 or 2, and may form a 5- to 6-membered ring together with the atoms to which they are bonded and with the carbonyl group,
allantoin, and salts thereof.

2. A SCCA-1 production inhibitor according to claim 1, wherein the carboxyamide derivative is one of the following: 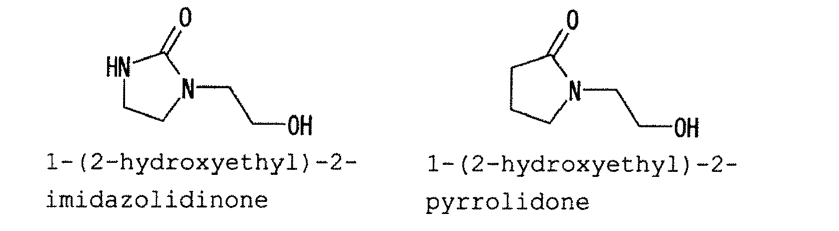 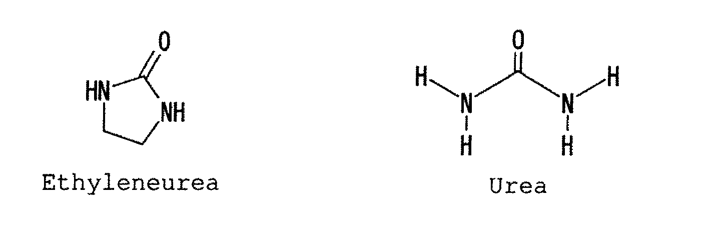 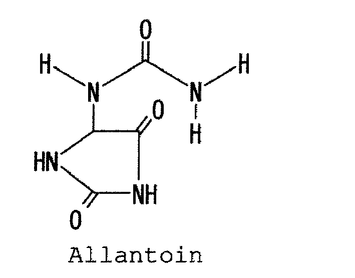 or a salt thereof.

3. A pharmaceutical composition for prevention and/or treatment of a disease associated with abnormal cell proliferation, comprising a carboxyamide derivative according to claim 1 or 2 and/or a salt thereof as an active ingredient.

4. A pharmaceutical composition for prevention and/or treatment of acanthosis, comprising a carboxyamide derivative according to claim 1 or 2 and/or a salt thereof as an active ingredient.

5. A pharmaceutical composition for treatment of a disease selected from the group consisting of malignant tumors (carcinoma) and precancerous conditions, such as basal cell carcinoma (BCC), squamous cell carcinoma (SCC), Bowen's disease, pilomatricoma (calcifying epithelioma), seborrheic keratosis and actinic keratosis (solar keratosis), lichen planus-like keratosis, benign
lichenoid keratosis, acrochordon (cutaneous tag), psoriatic conditions such as pustular psoriasis and psoriasis vulgaris, xeroderma pigmentosum (XP), atopic dermatitis, erythematosus including systemic lupus erythematosus (SLE) and discoid lupus erythematosus (DLE), porokeratosis, moles or verrucas such as inflammatory linear verrucous epidermal naevus (ILVEN), and benign keratosis such as hyperplasia, comprising a carboxyamide derivative according to claim 1 or 2 and/or a salt thereof as an active ingredient.
